# EUROPEAN PATENT APPLICATION

(11) **EP 1 430 902 A1**
(43) Date of publication of application: **23.06.2004**
(21) Application number: 02028574.8
(22) Date of filing: 20.12.2002
(51) Int. Cl.: A61K 38/21, C12Q 1/68, C12N 15/10, A61P 11/06

(54) **Pharmaceutical composition of interferon gamma with molecular diagnostics for the improved treatment of asthma bronchiale**

(71) Applicant: Mondobiotech Laboratories Anstalt, 9490 Vaduz (LI)
(72) Inventor: Bevec, Dorian, Dr., 6925 Gentilino (CH); Ziesche, Rolf, 1210 Wien (AT)
(74) Representative: Salgo, Reinhold Caspar, Dr.

(57) **Abstract**

The present invention relates to a novel pharmaceutical composition comprising interferon-y and a diagnostic array of candidate polynucleotides for the improved treatment of lung diseases, especially for all forms of asthma bronchiale. This invention describes the combination of molecular diagnosis and clinical therapy as a novel medication principle for reduction of mortality and improvement of disease management in asthma bronchiale.

## Description

The present invention relates to a novel pharmaceutical composition of compounds having the biological activity of interferon gamma (IFN-γ) in combination with a diagnostic array of candidate polynucleotides for the improved treatment of all forms of asthma bronchiale.

### BACKGROUND OF THE INVENTION

### Asthma bronchiale

The cell biology of human bronchial asthma is in many ways similar to the mechanisms underlying lung fibrosis. At early stages of bronchial asthma, the peripheral respiratory tract shows characteristic signs of fibrosis. A specific feature of bronchial asthma is the functional and reversible narrowing of the respiratory tract as a result of the fibroproliferative reaction. Patients with bronchial asthma of several years' duration have characteristic signs of a *chronic inflammation with a major wound healing reaction* and become increasingly unresponsive to anti-inflammatory medication. Our molecular investigations demonstrated that chronic inflammation in asthmatics - as in patients with lung fibrosis - is characterised by an IFN-γ deficiency.
Currently, none of the marketed treatments addresses airway remodeling.
According to the WHO, the prevalence of bronchial asthma in US, Europe and Japan should reach approx. 41.5 million patients, of whom 10% show severe asthma. There are dramatic increase rates regarding asthma, especially in children.

As IFN-γ is a pleiotropic mediator in immune responses to various infections, it cannot be considered as a generally applicable therapeutic agent without careful, molecular gene expression analyses of the patients. Therefore, we consider thera-nostics - the combination of a therapeutic agent and molecular diagnostics as a key success factor in managing severe asthma bronchiale.
Even if the causative agents were known, at present organ fibrosis cannot be sufficiently treated with any medication. Millions of people are dying from slow destruction of vital organ systems owing to pathological restructuring of functional organ tissue. This relentless process is known as fibrosis or tissue remodeling that is primarily due to fibroproliferative mechanisms.
The fibroproliferative reaction concerns all organs of the body. In the bronchi it is known as bronchial asthma, in the gas-exchanging lung tissue it is known as lung fibrosis, in the liver as cirrhosis, in the kidney as glomerulosclerosis, in the general circulation as arteriosclerosis and coronary artery sclerosis, and in the pulmonary circulation as pulmonary hypertension.
These conditions are collectively known as fibroproliferative diseases. In fibrosis, healthy tissue is progressively replaced by components of the connective and supporting tissue of the human body. This process is based on the pathologically accelerated growth rate of tissue cells, which would normally accomplish regular wound healing. Thus, fibroproliferative diseases may be defined as uncontrollably accelerated wound healing. In fibrosis, the replacement of functional organ tissue continues until complete loss of organ function occurs. The currently available modes of diagnosis and treatment for all fibroproliferative diseases are inadequate.

### Interferon gamma (IFN-γ)

IFN-γ is a naturally glycoprotein cytokine, acting synergistically with other cytokines to exert its wide ranging effects. IFN-γ is a naturally occurring glycoprotein having a molecular weight of about 17.000 which can be commercially produced today also by recombinant techniques.

For improvement of the pharmacokinetic features, IFN-γ can be modified by pegylation to obtain PEG-IFN-γ. In addition to injection techniques, IFN-γ could be administered by inhalation to the lungs to diminish undesirable systemic side effects. IFN-γ exhibits antiviral activity and together with other cytokines it plays an pivotal immunoregulatory role within the human immune system (an immunostimulating or an immunosuppressive effect, dependent on cell activation and location). Effects of IFN-γ include the induction of MHC class II antigens, macrophage activation, increased immunoglobulin production from B lymphocytes and enhanced NK cell activity.
The general antifibrotic properties of IFN-γ are well-documented *(Lortat-Jacob H, Kleinman HK, Grimaud JA. High-affinity binding of interferon-gamma* to *a basement membrane complex (matrigel). J Clin Invest. 1991; 87: 878-83.; Narayanan AS, Whithey J, Souza A, Raghu G. Effect of gamma-interferon on collagen synthesis by normal and fibrotic human lung fibroblasts. Chest. 1992 May;101(5):1326-31; Hjelmeland LM, Li JW, Toth CA, Landers MB 3d. Antifibrotic and uveitogenic properties of gamma interferon in the rabbit eye. Graefes Arch Clin Exp Ophthalmol. 1992;230(1):84-90; Hyde DM, Henderson TS, Giri SN, Tyler NK, Stovall MY. Effect of murine gamma interferon on the cellular responses to bleomycin in mice. Exp Lung Res. 1988;14(5):687-704.).* It has already been demonstrated that IFN-γ causes a reduced expression of TGF-β₁ together with a reduction in the amount of fibrosis *(Giri SN, Hyde DM, Marafino BJ Jr. Ameliorating effect of murine interferon gamma on bleomycin-induced lung collagen fibrosis in mice. Biochem Med Metab Biol. 1986 Oct;36(2):194-7.).* When using IFN-γ under clinical conditions, the transcription of TGF-β₁ and that of CTGF are significantly diminished after six months of therapy *(Ulloa L, Doody J, Massague J. Inhibition of transforming growth factor-beta*/*SMAD signalling by the interferon-gamma*/*STAT pathway. Nature 1999; 397: 710-3.).* The simultaneous improvement of lung function in individuals suffering from idiopathic pulmonary fibrosis receiving IFN-γ provides additional support for the hypothesis that the mesenchymal activation in individuals with lung fibrosis depends, at least in part, on the continuous overexpression of TGF-β₁ and CTGF *(Ziesche R, Hofbauer E, Wittmann K, Petkov V, Block LH. A preliminary study of long-term treatment with interferon gamma-1b and low-dose prednisolone in individuals with idiopathic pulmonary fibrosis. N Engl J Med. 1999; 341: 1264-9; EP0795332A2.).* Moreover, these results suggest that an acquired deficiency of IFN-γ may be one reason for the exaggerated wound healing process characteristic of progressive organ fibrosis.
IFN-γ was shown to be the first clinically applicable anti-fibrotic drug. As any endogenous substance, IFN-γ has pleiotropic effects dependent on the existing conditions within the body. In case of an exaggerated, yet non- or low-inflammatory wound healing, such as in asthma bronchiale, the drug exerts powerful highly beneficial anti-fibrotic properties. However, in the presence of accompanying infections, such as chronic bacterial, viral or fungal infections, the drug will add to the inflammatory process and even reinforce, by intensifying tissue repair, the fibrotic process.

Thus, for a safe and effective use of the drug, it is necessary to molecularly measure the state of tissue response prior to application of the drug. The absence of the mRNA transcription of the gene for IFN-γ in the presence of intensified transcription of factors of wound healing may serve as the first vague discriminator between fibrosis as a result of deregulated wound healing itself, versus fibrosis as a result of intensified inflammation. A medication which combines the molecular diagnosis (transcription analysis in asthma bronchiale patients compared to control populations - healthy individuals or patients suffering from different lung diseases - with a distinct quantitative, specific expression difference to those) with proven effectiveness of a antifibrotic drug like IFN-γ under clinical conditions provides the essential step for the first bio-medical based treatment of this multifunctional disease.

To sum up, the real effectiveness of IFN-γ therapy mainly depends on four conditions:
(a) the proof of IFN-γ deficiency in pulmonary tissue;
(b) the control of infections or other inflammatory conditions, either pre-existing or acquired during therapy;
(c) the gene expression profiling of the patients suspected to have asthma bronchiale;
(d) the duration of disease-related symptoms.

Clinical experience suggests that repeated phases of increased inflammation due to a large variety of infectious agents are the most common reason for a failure of IFN-γ in the treatment of lung diseases. Thus, clinical and molecular control of inflammation and infections, especially in the peripheral bronchi is crucial for a success of an anti-fibrotic treatment with IFN-γ.

Thus it is goal of the work of the present invention to improve the treatment of lung diseases, especially asthma bronchiale with novel pharmaceuticals using IFN-γ in pharmaceutical composition with a diagnostic array of candidate polynucleotides (genechip).

### Disease-related gene expression profiles

With the completion of the Human Genome Project sequencing, biomedical research will be revolutionised by the ability to carry out investigations on a genome wide scale:
Arch Ophthalmol 2001 Nov;119(11):1629-34
   Microarray analysis of gene expression in human donor corneas.
Jun AS et al.
   Ann Surg 2001 Dec;234(6):769-78; discussion 778-9 Identification of disease-specific genes in chronic **pancreatitis** using DNA array technology. Friess H et al.
DNA Cell Biol 2001 Nov;20(11):683-95
   A gene expression profile of **Alzheimer's disease.** Loring JF et al.
Pharmacogenomics J 2001;1(4):272-87
   Molecular classification of **psoriasis** disease-associated genes through pharmacogenomic expression profiling. Oestreicher JL et al.
J Neurosci 2002 Apr 1;22(7):2718-29
   Gene expression profiling reveals alterations of specific metabolic pathways in **schizophrenia**. Middleton FA et al.
J Natl Cancer Inst 2002 Apr 3;94(7):513-521
   Osteopontin Identified as Lead Marker of **Colon Cancer** Progression, Using Pooled Sample Expression Profiling. Agrawal D et al.
Blood 2002 Mar 15;99(6):2037-44
   Gene expression profiling identifies significant differences between the molecular phenotypes of bone marrow-derived and circulating human CD34(+) **hematopoietic stem cells**. Steidl U et al.
Invest Ophthalmol Vis Sci 2002 Mar;43(3):603-7
   Gene expression profile of native human **retinal pigment epithelium.**
   Buraczynska M et al.
Cancer Res 2002 Feb 15;62(4):1184-90
   Molecular classification of **head and neck squamous cell carcinoma** using cDNA microarrays.
   Belbin TJ et al.
Oncogene 2002 Feb 21;21(9):1346-58
   Identification of genes regulated by **dexamethasone in multiple myeloma cells** using oligonucleotide arrays.
   Chauhan D et al.
Genome Res 2002 Feb;12(2):281-91
   In vivo regulation of human **skeletal muscle** gene expression **by thyroid hormone.**
   Clement K et al.
Cancer Res 2002 Feb 1;62(3):819-26
   Relationships and differentially expressed genes among **pancreatic cancers** examined by large-scale serial analysis of gene expression.
   Ryu B et al.
J Vasc Surg 2002 Feb;35(2):346-55
   Differential gene expression in human **abdominal aorta:** aneurysmal versus occlusive disease.
   Armstrong PJ et al.
Lancet 2002 Jan 12;359(9301):131-2
   Identification of high risk **breast-cancer** patients by gene expression profiling.
   Ahr A et al.
Nature 2002 Jan 31;415(6871):530-6
   Gene expression profiling predicts clinical outcome of **breast cancer**.
   van 't Veer LJ et al.
Oncogene 2002 Jan 17;21(3):475-8
   Global gene expression profiling in **Barrett's esophagus and esophageal cancer:** a comparative analysis using cDNA microarrays.
   Selaru FM et al.
Mol Carcinog 2002 Jan;33(1):25-35
   Gene expression analysis of **prostate cancers**.
   Luo JH et al.
Nature 2002 Jan 24;415(6870):436-42
   Prediction of central **nervous system embryonal tumour** outcome based on gene expression.
   Pomeroy SL et al.
FASEB J 2002 Jan;16(1):61-71
   Gene profiling reveals unknown enhancing and suppressive **actions of glucocorticoids on immune cells.**
   Galon J et al.
Cancer Res 2002 Jan 1;62(1):233-40
   Global gene expression analysis of **gastric cancer** by oligonucleotide microarrays.
   Hippo Y et al.
Nat Med 2002 Jan;8(1):68-74
   Diffuse large **B-cell lymphoma** outcome prediction by gene-expression profiling and supervised machine learning.
   Shipp MA et al.
Mol Carcinog 2002 Feb;33(2):113-24
   Analysis of differentially expressed genes in **hepatocellular carcinoma** using cDNA arrays.
   Goldenberg D et al.
J Dent Res 2002 Feb;81(2):89-97
   DNA hybridization arrays for gene expression analysis of human **oral cancer**.
   Todd R, Wong DT
Inflamm Bowel Dis 2002 Mar;8(2):140-57
   Application of genome-wide gene expression profiling by high-density DNA arrays to the treatment and study of **inflammatory bowel disease**.
   Warner EE, Dieckgraefe BK.
Invest Ophthalmol Vis Sci 2002 Mar;43(3):603-7
   Gene expression profile of native human **retinal pigment epithelium.**
   Buraczynska M et al.
Adv Cancer Res 2002;84:1-34
   Gene expression in inherited **breast cancer**.
   Hedenfak IA et al.

In addition, a significant number of patent applications are addressing the described molecular techniques in medical indications, for example:
AU3479902 Gene expression profiling of primary breast carcinomas using arrays of candidate genes
AU1160002 Gene expression profiling of antidepressant action in the brain
US2002102553 Molecular markers for the diagnosis of alzheimer's disease
WO02057496 GENE EXPRESSION PROFILING OF ENDOTHELIUM IN ALZHEIMER'S DISEASE
WO02057414 LEUKOCYTE EXPRESSION PROFILING
EP1226277 A METHOD FOR HIGH-DENSITY MICROARRAY MEDIATED GENE EXPRESSION PROFILING
EP1221618 Method for diagnosing allergic diseases
WO0246467 GENE EXPRESSION PROFILING OF PRIMARY BREAST CARCINOMAS USING ARRAYS OF CANDIDATE GENES
WO0244732 METHOD FOR DIAGNOSING ALLERGIC DISEASES
WO0238729 GENE MONITORING AND GENE IDENTIFICATION USING cDNA ARRAYS
WO0229116 GENE EXPRESSION PROFILING OF ANTIDEPRESSANT ACTION IN THE BRAIN
CA2318984 TUMOR TISSUE MICROARRAYS FOR RAPID MOLECULAR PROFILING
   US2002029391 EPITOPE-DRIVEN HUMAN ANTIBODY PRODUCTION AND GENE EXPRESSION PROFILING
US2002009767 Frozen tissue microarrays and methods for using the same
EP1171766 SYSTEM AND METHOD FOR MANAGING AND PRESENTING INFORMATION DERIVED FROM GENE EXPRESSION PROFILING
WO0173134 GENE PROFILING ARRAYS
AU2468501 Gene expression profiling of inflammatory bowel disease
AU1201301 A method for high-density microarray mediated gene expression profiling

Gene expression technology is gaining increasingly widespread use as a means to determine the expression of potentially all human genes at the level of messenger RNA. Gene specific sequences (oligo-, polynucleotides or cDNAs) are immobilised on arrays, hybridised with complex probes represented by a mixture of cDNAs of respective samples from human biopsies and other human materials (reversely transcribed from messenger RNA), and labelled with different dyes. Hybridised slides are analysed with sensitive scanning methodologies. Genechips containing gene sequences deliver fast and excellent overview of the expression pattern of the biological samples. Thus, the genome-wide gene expression analysis provides new insights into causes of disease, and elucidates the as yet unknown biological gene expression variations between seemingly similar diseases in defined detection ranges, leading to a new classification system valuable in accurate diagnosis.

Important applications include:
(a) development of a more global understanding of the qualitative and quantitative gene expression profiles that contribute to disease processes;
(b) discovery of new diagnostic and prognostic indicators and biomarkers of therapeutic response;
(c) identification and validation of new molecular targets for drug development;
(d) provision of an improved understanding of the molecular mode of action during lead identification and optimisation;
(e) designing more specific and efficient treatment strategies;
(f) prediction of potential side-effects during development and toxicology studies;
(g) confirmation of molecular modes of action during hypothesis-testing clinical trials;
(h) identification of genes involved in conferring drug sensitivity and resistance;
(i) prediction of patients most likely to benefit from the drug and use in general pharmacogenomic studies.

As a result of further technological improvements, array-based genechips will become essential part for clinical interventions in identifying and quantifiying individual gene-expression patterns for purposes of patient-oriented therapy, and in establishing a method for predicting efficacy of drugs for individual patients, given the functional complexity of most diseases, especially those involving fibroproliferative processes. Considering the fact that inflammatory diseases reflect qualitative and quantitative changes in the activity of certain genes and/or gene clusters, the molecular assessment will allow for a higher specificity and efficacy of medical treatments and will be considered integral part of the therapeutic composition.
To achieve this goal, new molecular markers and markers of progression are needed for improved staging and for better assessment of diagnosis and treatment of complex diseases. Gene expression profiling techniques in combination with therapeutics offer the opportunity to discover such pathophysiologically relevant markers of disease.

In the context of this disclosure, a number of terms shall be utilized.
The term "polynucleotide" refers to a polymer of RNA or DNA that is single-stranded, optionally containing synthetic, non-natural or altered nucleotide bases. A polynucleotide in the form of a polymer of DNA may be comprised of one or more segments of cDNA, genomic DNA or synthetic DNA.
The term "subsequence" refers to a sequence of nucleic acids that comprises a part of a longer sequence of nucleic acids. The term "immobilized on a support" means bound directly or indirectly thereto including attachment by covalent binding, hydrogen bonding, ionic interaction, hydrophobic interaction or otherwise.

An aspect of the invention relates to polynucleotide arrays, which allows to qualitatively and quantitatively study mRNA expression levels of selected candidate genes in human materials.

Polynucleotide or DNA arrays consist of large numbers of DNA molecules spotted in a systematic order on a solid support or substrate such as a nylon membrane, glass slide, glass beads or a silicon chip. Depending on the size of each DNA spot on the array, DNA arrays can be categorized as microarrays (each DNA spot has a diameter less than 250 microns) and macroarrays (spot diameter is grater than 300 microns). When the solid substrate used is small in size, arrays are also referred to as DNA chips. Depending on the spotting technique used, the number of spots on a glass microarray can range from hundreds to tens of thousands.

DNA microarrays serve a variety of purposes, including gene expression profiling, de novo gene sequencing, gene mutation analysis, gene mapping and genotyping. cDNA microarrays are printed with distinct cDNA clones isolated from cDNA libraries. Therefore, each spot represents an expressed gene, since it is derived from a distinct mRNA.
Typically, a method of monitoring gene expression involves providing
(1) a pool of sample polynucleotides comprising RNA transcripts of target genes or nucleic acids derived from the RNA transcripts;
(2) hybridizing the sample polynucleotides or nucleic acids derived from the RNA transcripts to an array of probes (for example, polynucleotides obtained from a polynucleotide library including control probes, and
(3) detecting the hybridized double-stranded polynucleotides/nucleic acids. The label used to mark polynucleotide samples is selected from the group consisting of radioactive, colorimetric, enzymatic, molecular amplification, bioluminescent or fluorescent label.

The invention relates also to any polynucleotide library as previously described wherein said polynucleotides are immobilized on a solid support in order to form a polynucleotide array.
Preferably the support is selected from the group consisting of a nylon membrane, glass slide, glass beads, or a silicon chip.
The invention relates to a polynucleotide library useful in the molecular characterization of asthma bronchiale, the library including a pool of polynucleotide sequences or subsequences thereof wherein the sequences or subsequences are either underexpressed or overexpressed in asthma bronchiale diseased cells when compared to non-asthma cells. Preferably, a set of sequences is selected from oligo- or polynucleotide probes having a sequence defined by, or correlated to, or derived from the group of genes consisting of candidate genes indicated in the following list, representing increased gene expression levels of asthma bronchiale cells versus cells from patients with a different lung disorder:
karyopherin alpha 2 (RAG cohort 1, importin alpha 1); U28386.1
vesicle-associated membrane protein 3 (cellubrevin); BC003570.1
TIA1 cytotoxic granule-associated RNA-binding protein; M77142.1
fatty-acid-Coenzyme A ligase, long-chain 2; D10040.1
protein tyrosine phosphatase, non-receptor type 12; D13380.1
transcrption factor IIIA-interacting protein; NM_021980.1
a disintegrin and metalloproteinase domain 10; AF009615.1
tumor necrosis factor (ligand) superfamily, member 10; U37518.1
A kinase (PRKA) anchor protein 2; AB023137.1
lysosomal-associated membrane protein 2; J04183.1
3-phosphoadenosine 5-phosphosulfate synthase 2; AF150754.2
caveolin 2; BC005256.1
membrane metallo-endopeptidase (neutral endopeptidase, enkephalinase, CALLA, CD10); J03779.1
ATP-binding cassette, sub-family A (ABC1), member 1; AF165281.1
adenylate cyclase 7; D25538.1
prenylcysteine lyase; AF181490.1
nebulette; NM_006393.1
KIAA0161 gene product; D79983.1
KIAA0680 gene product; AB014580.1
malic enzyme 1, NADP(+)-dependent, cytosolic; NM_002395.2
Friend leukemia virus integration 1; BC001670.1
G protein-coupled receptor kinase 5; L15388.1
frizzled (Drosophila) homolog 1; AB017363.1
endothelin receptor type A; L06622.1
alpha integrin binding protein 63; AB023234.1
dihydropyrimidine dehydrogenase; U20938.1
Wnt inhibitory factor-1; AF122922.1
KIAA0716 gene product; AB018259.1
retinitis pigmentosa 2; NM_006915.1
E3 ubiquitin ligase SMURF2; AF301463.1
oxidative 3 alpha hydroxysteroid dehydrogenase; retinol dehydrogenase; 3-hydroxysteroid epimerase (RODH); NM_003725.1
integrin, alpha 4 (antigen CD49D, alpha 4 subunit of VLA-4 receptor); NM_000885.2
klotho; AB005142.1
carboxypeptidase M; NM_001874.1
toll-like receptor 3; U88879.1
interleukin 18 (interferon-gamma-inducing factor); D49950.1
interleukin 18 receptor 1; U43672.1
ubiquitin specific protease 9; NM_004654.2
TXK tyrosine kinase; L27071.1
Ste20-related serine/threonine kinase; D86959.1
Sciellin; AF045941.1
pleiomorphic adenoma gene-like 1; U81992.1
UDP-N-acetylglucosamine:polypeptide-N-acetylglucosaminyl transferase; U77413.1
nuclear receptor coactivator 3; AF036892.1
glycoprotein M6B; AF016004.1
TRAF family member-associated NFKB activator; U59863.1
GATA-binding protein 6; U66075.1
potassium inwardly-rectifying channel, subfamily J, member 15; U73191.1
RecQ protein-like (DNA helicase Q1-like); BC001052.1
tissue factor pathway inhibitor (lipoprotein-associated coagulation inhibitor); AF021834.1
calcitonin receptor-like; U17473.1
transcription factor 8 (represses interleukin 2 expression); U12170.1
UDP-Gal:betaGlcNAc beta 1,3-galactosyltransferase, polypeptide 3; AB050856.1
transposon-like element; AL567779
DKFZp434I0812; AI057093
sperm associated antigen 9; AK023512.1
protein tyrosine phosphatase, receptor type, C; AI809341
KIAA0033 protein; AU153138
DKFZP564M1416 protein; BF966021
KIAA0197 protein; D83781.1
hypothetical protein FLJ22028; AI814728
hypothetical protein DKFZp762B226; AK001821.1
Hmob33 protein; AI377497
synaptojanin 1; AB020717.1
Highly similar to A59252 myosin heavy chain, nonmuscle; AI382123
clone 23664 and 23905 sequence; AI769688
DKFZp586B211; AA045174
Novel human gene mapping to chomosome 13; N80918
chloride intracellular channel 2; NM_001289.2
ATPase, Class VI, type 11A; BF439472
KIAA0580 protein; AB011152.1
DKFZp586F1323; AI743740
v-ral simian leukemia viral oncogene homolog A (ras related); M29893.1
synaptosomal-associated protein, 23kD; Y09568.1
collagen, type IV, alpha 3 (Goodpasture antigen); NM_000091.1
hypothetical protein KIAA1164; AU121431
hypothetical protein FLJ20275; AW270932
Homo sapiens mRNA from chromosome 5q21-22, clone FBR89; AB002438.1
KIAA1043 protein; AB028966.1
fragile X mental retardation 1; AA830884
HEMBA1003278; AU144887
KIAA1033 protein; AK001657.1
up-regulated by BCG-CWS; AL049963.1
clone COL05634; AK025152.1
clone LNG01738; AK026679.1
collagen, type IV, alpha 3 (Goodpasture antigen) alternatively spliced mRNA; U02519.1
T cell receptor delta; X06557.1
Highly similar to IFT2 HUMAN INTERFERON-INDUCED PROTEIN WITH TETRATRICOPEPTIDE REPEATS 2; BE888744
CLONE=IMAGE:1269464; AA721025
CLONE=IMAGE:1680846; AI086530
CLONE=IMAGE:3859303; BF032808
hypothetical protein FLJ12910; NM_024573.1
hypothetical protein FLJ13164; NM_022776.1
hypothetical protein FLJ20331; NM_017768.1
hypothetical protein FLJ22029; NM_024949.1
pleckstrin homology domain-containing, family A (phosphoinositide binding specific) member 1; AF286160.1
zinc finger protein 331; AF251515.2
hypothetical protein FLJ14281; NM_024920.1
hypothetical protein FLJ23516; NM_024539.1
hypothetical protein FLJ22690; NM_024711.1
dickkopf (Xenopus laevis) homolog 2; AF177395.1
clone=IMAGE-2075682; AI832249
clone=IMAGE-290072; N64681
clone=IMAGE-2112146; AI400621
KIAA0823 protein; AB020630
protein-tyrosine-phosphatase D1; X79510
lok for protein kinase; AB015718
oxidative 3 alpha hydroxysteroid dehydrogenase; U89281
KIAA0615 protein; AB014515
KIAA1466 protein; AI765383
CLONE=IMAGE:2337182; AI694562
hypothetical protein FLJ20059; AW006750
sec61 homolog; AI719730
clone 24421 mRNA sequence; BE881590
KIAA1598 protein; AU157109
mannosidase, alpha, class 1A, member 1; BG287153
Claudin-18; AF221069.1
colon carcinoma related protein; AF099505.1
hypothetical protein FLJ23151; NM_024772.1
serumglucocorticoid regulated kinase-like; AF169035.1
hyaluronic acid receptor; AF127670.2
hypothetical protein FLJ20716; NM_017938.1
cytochrome P450 retinoid metabolizing protein; AF252297.1
hypothetical protein FLJ23045; NM_024704.1
TMTSP for transmembrane molecule with thrombospondin module; AB044385.1
macrophage scavenger receptor 1; AI299239
transforming growth factor β₁; NM_000660
wherein each oligo- or polynucleotide probe is obtainable on the basis of structural information mediated by the sequence data provided by the respective accession number set out for each candidate gene.

Yet another set of sequences is selected from oligo- or polynucleotide probes having a sequence defined by, or correlated to, or derived from the group of genes consisting of candidate genes indicated in the following list, representing decreased gene expression levels of asthma bronchiale cells versus cells from patients with different lung disorder:
Alu-Sq subfamily; U14573
mRNA clone 9112; X57348
prostate differentiation factor; AF003934.1
G protein-coupled receptor 87; NM_023915.1
PROCOLLAGEN ALPHA 1(III) CHAIN PRECURSOR; AU144167
hypothetical protein FLJ13556; BE300252
Tubulin, alpha; AL581768
collagen, type III, alpha 1; AF130082.1
tubulin alpha 1; BC006481.1
lectin, galactoside-binding, soluble, 2 (galectin 2); M87842.1
eukaryotic translation elongation factor 1 alpha 1-like 14; NM_001403.1
eukaryotic translation elongation factor 1 alpha 1; NM_001402.1
annexin A8; NM_001630.1
collagen, type I, alpha 2; NM_000089.1
collagen, type I, alpha 1; NM_000088.1
hexabrachion (tenascin C, cytotactin); M55618.1
ribosomal protein L41; NM_021104.1
insulin-like growth factor binding protein 7; L19182.1
interferon-gamma: X87308
wherein each oligo- or polynucleotide probe is obtainable on the basis of structural information mediated by the sequence data provided by the respective accession number set out for each candidate gene.
For example, suitable sequences which may be used for oligonucleotide design may be downloaded from the NCBI GenBank (http://www.ncbi.nlm.gov/GenBank/index.html) using the Accession number listed behind the gene name.
All accession numbers give access to mRNA sequences; since the cDNA of the patients is reverse transcribed, the oligonucleotide have to sense strand and are therefore identical with the mRNA sequence.
Most preferably the pool of polynucleotide sequences or subsequences correspond substantially to the polynucleotide sequences useful in qualitative and quantitative differentiating a normal cell, or a cell from a patient suffering from a different fibrosing lung disease, from a cell of an patient with asthma bronchiale.
The invention concerns the part of pharmaceutical composition for detecting differentially expressed polynucleotide sequences which are correlated with asthma bronchiale, said part comprising: a) obtaining a polynucleotide sample from a patient; and b) reacting the sample polynucleotide obtained in step (a) with a probe immobilized on a solid support
wherein said probe comprises any of the polynucleotide sequences of the libraries previously described or an expression product encoded by any of the polynucleotide sequences of said libraries and c) detecting the reaction product of step (b) as a prerequisite for a subsequent administration of suitable drug.

The invention relates also to the part of pharmaceutical composition detecting differentially expressed polynucleotide sequences of the invention wherein the amount of reaction product of step (c) is compared to control samples.

The detection of differentially expressed polynucleotide sequences is used for detecting, diagnosing, staging, monitoring, prognosticating, preventing or treating conditions associated with asthma bronchiale.

The detection of differentially expressed polynucleotide sequences is particular useful wherein the product encoded by any of the polynucleotide sequences or subsequences is involved in a receptor-ligand reaction on which detection is based.
The invention relates also to detecting differentially expressed polynucleotide sequences previously described
wherein the sample has been treated with the anti-asthma drug to be screened.
The invention also relates to a library of polynucleotides comprising a population of polynucleotide sequences overexpressed or underexpressed in cells derived from asthma bronchiale patients.
A particular embodiment of the invention relates to a polynucleotide library of corresponding substantially to any combination of at least one polynucleotide sequence selected among those included in each one of predefined polynucleotide sequences sets mentioned above.

The invention relates to polynucleotide libraries comprising at least one polynucleotide selected among those included in at least 50%, preferably 75% and more preferably 100% of said predefined sets, allowing to obtain a discriminating gene pattern, namely to distinguish between normal individuals and patients suffering from asthma bronchiale.

Polynucleotide sequences library useful for the realization of the invention can comprise also any sequence comprised between 3'-end and 5'-end of each polynucleotide sequence sets as defined above, allowing the complete detection of the implicated genes.

The invention relates also to a polynucleotide library useful to differentiate a normal cell from a cell of asthma bronchiale patients wherein the pool of polynucleotide sequences or subsequences correspond substantially to any combination of at least one polynucleotide sequence selected among those included in each one of predefined polynucleotide sequences sets indicated above, useful in differentiating a normal cell from a cell from asthma bronchiale patients.

Differences in gene expression are accepted as different when the signals from patient material are at least two fold lower or higher than those from comparative material, be it from healthy volunteers material or from other diseased material. This threshold is commonly accepted for the evaluation of expression arrays.
The invention additionally provides a method for identifying gene expression or genomic DNA of infective agents including bacteria (Mycobacterium spec., Mycoplasma spec., Staphyllococcus aureus, treptococcus spec., Borrelia, Treponema pallidum, Leptospira interrogans, Campylobacter jejuni, fetus; Escherichia coli, EPEC, ETEC, EIEC, EHEC Salmonella enterica, Yersinia enterocolitica, Aeromonas spec., Campylobacter fetus, Moraxella catarrhalis, Moraxella catarrhalis, Brucella spec., Toxoplasma, Salmonella enterica, Shigella spec., Yersinia enterocolitica, Vibrio cholerae, Pseudomonas aeruginosa, Burkholderia cepacia, Stenotrophomonas maltophilia, Acinetobacter baumanii, Acitenobacter calcoaceticus, Klebsiella, Enterobacter, Citrobacter, Proteus, Serratia, Morganella, Providencia, Cardiobacterium hominis, Eikenella corrodens, Gardnerella vaginalis, Calymmatobacterium granulomatis, Bacteriodes, Porphyromonas, Prevotella, Fusobacterium, Rickettsia prowazekii, Bartonella bacilliformis, Bartonella henselae and Chlamydia spec.), yeasts, fungi, or viruses (retroviruses, adenoviruses, hepadnaviruses, herpesviruses, influenza viruses, paramyxoviruses) as cellular parasites in cells from patients with asthma bronchiale.

### SUMMARY OF THE INVENTION

It was surprisingly found that a pharmaceutical composition of interferon gamma together with a gene expression analysis of the patients with lung diseases results in a faster and more successful treatment of lung diseases, preferably asthma bronchiale. Thus, the invention relates to new pharmaceutical compositions and pharmaceutical kits comprising interferon gamma and a disease-oriented gene expression analysis.

To sum up and in more detail, the invention relates to the following topics:
- A pharmaceutical composition comprising
   (i) interferon gamma (IFN-γ), or pegylated (PEG) IFN-γ), optionally together with a pharmaceutically acceptable carrier, diluent or excipient,
   (ii) a gene expression analysis of patients with asthma bronchiale (genechip);
- a corresponding pharmaceutical composition, comprising additionally a glucocorticoid compound;
- a corresponding pharmaceutical composition, wherein the medication is selected from the following group:
   a pharmaceutical kit comprising
      (i) a package comprising IFN-γ, or PEG-IFN-γ,
      (ii) a package comprising a gene expression analysis (genechip) of the patient with lung disease;
- a corresponding pharmaceutical kit further comprising a package comprising a glucocorticoid compound;
- a method or a use of a combination of a pharmaceutical composition or a pharmaceutical kit according as specified for the manufacture of a medicament for the treatment of asthma bronchiale;
- a corresponding method or use, wherein IFN-γ is administered to the individual in a single dose amount varying from 2.0 to 3.0 µg / kg body weight;
- a corresponding method or use, wherein the glucocorticoid compound is administered to the individual in a single dose amount varying from 100 to 150 µg / kg body weight;
- a corresponding method or use, wherein IFN-γ is administered to the individual by inhalation after a gene expression analysis.

### DETAILED DESCRIPTION

Suitable compounds which have the therapeutic effect within the combination according to the invention, are, besides interferon gamma, or pegylated interferon gamma, compounds which have the same, but also enhanced, biological activity of interferon gamma, or pegylated interferon gamma in combination with the gene expression analysis of diseased patients.
The invention includes also derivatives, analogues, homologues, fusion proteins, stabilized forms, etc., of the disclosed drugs, as more specified above, which have the same biological activity as interferon gamma.
The term **"same biological activity"** means herein the same substantial biological, physiological or therapeutic activity or functionality, which however can be quantitatively enhanced or reduced compared with the relevant properties of said drugs.
The term **"stabilized form"** means a derivative or analogue wherein the parent drug was altered in order get more stability and increased half-life in blood and serum. Polypeptides and proteins may be protected against proteolysis by the attachment of chemical moieties. Such attachment may effectively block the proteolytic enzyme from physical contact with the protein backbone itself, and thus prevent degradation. Polyethylene glycol is one such chemical moiety which has been shown to protect against proteolysis *(Sada, et al., J. Fermentation* Bioengineering *71: 137-139, 1991).* In addition to protection against proteolytic cleavage, chemical modification of biologically active proteins has been found to provide additional advantages under certain circumstances, such as increasing the stability and circulation time of the therapeutic protein and decreasing immunogenicity. *(US. 4,179,337; Abuchowski et al., Enzymes as Drugs.; J.S. Holcerberg and J. Roberts, eds. pp. 367-383, 1981; Francis, Focus on Growth Factors 3: 4-10; EP 0 401 384).* The addition of polyethylene glycol increases stability of the peptides and polypeptides of this invention at physiological pH as compared to non-pegylated compounds. The pegylated polypeptide /protein is also stabilized with regard to salts.
The term **"fusion protein"** means a compound, especially a stabilized form, consisting of a polypeptide according to the invention, preferably interferon gamma, which is fused to another peptide or protein.
The term **"pharmaceutical kit"** means a package comprising two or more packages or containers containing one or more, preferably one pharmaceutically active compound or agent and a gene expression analysis device, wherein the agent or compound in said packages or containers are administered to an individual after gene expression analysis is performed. The pharmaceutical compositions according to the invention comprising interferon gamma and a gene expression analysis as defined above and below can be used as medicament for the treatment of an individual.
The term "**individual**" preferably refers to mammals, especially humans.

The compound according to this invention, IFN-γ, is used in a pharmaceutical formulations, comprising, as a rule, a pharmaceutically acceptable carrier, excipient or diluents. Techniques for the formulation and administration of the compounds of the present invention may be found in "Remington's Pharmaceutical Sciences" Mack Publishing Co., Easton PA.
As used herein, the term **"pharmaceutically acceptable carrier"** means an inert, non toxic solid or liquid filler, diluent or encapsulating material, not reacting adversely with the active compound or with the individual, or any other formulation such as tablets, pills, dragees, capsules, gels, syrups, slurries, suspensions and the like. Suitable, preferably liquid carriers are well known in the art such as sterile water, saline, aqueous dextrose, sugar solutions, ethanol, glycols and oils, including those of petroleum, animal, vegetable, or synthetic origin, for example, peanut oil, soybean oil and mineral oil. Tablets and capsules for oral administration contain conventional excipients such as binding agents, fillers, diluents, tableting agents, lubricants, disintegrants, and wetting agents. The tablets may be coated according to methods well known in the art.
The formulations according to the invention may be administered as unit doses containing conventional non-toxic pharmaceutically acceptable carriers, diluents, adjuvants and vehicles which are typical for parenteral administration.
The term **"parenteral"** includes herein subcutaneous, intravenous, intra-articular and intratracheal injection and infusion techniques. Parenteral compositions and combinations are most preferably administered intravenously either in a bolus form or as a constant fusion according to known procedures.

Also other administrations such as oral administration or administration by inhalation or nasal spray are also object of the invention. Inhalation of vapors containing interferon gamma as specified is also a preferred way of administration. For inhalations the compound according to the invention is preferably brought in an aerosol form. Aerosols and techniques to make them are well known in the art. Aerosols applicable by inhalers containing a polypeptide of the invention, for example, interferon gamma are preferred if direct pulmonary symptoms have to be treated.

Unit doses according to the invention may contain daily required amounts of the compound according to the invention, or sub-multiples thereof to make up the desired dose. The optimum therapeutically acceptable dosage and dose rate for a given individual (mammals, including humans) depends on a variety of factors, such as the activity of the specific active material employed, the age, body weight, general health, sex, diet, time and route of administration, rate of clearance, enzyme activity, the object of the treatment, i. e., therapy or prophylaxis and the nature of the disease to be treated.
Therefore, in the pharmaceutical compositions according to the invention for the therapy of an individual, a pharmaceutical effective daily dose of the respective compound in said composition is:
Interferon gamma (IFN-γ): It could be shown that interferon-γ is effective in the combination therapy according to the invention in a dose of 1.0 - 5.0 µg / kg body weight, preferably 2.0 - 3.0 µg / kg body weight, 1- 5 times per week. The above-indicated single dosages of interferon-γ are administered parenteral, preferably subcutaneously to the patient.

The doses of glucocorticoids which can be administered optionally together with IFN-γ to an individual vary according to the invention from 10 - 100 mg / single dose and more preferably from 15 - 80 mg, which corresponds to approximately 100 - 350 µg /kg body weight, preferably 100-150 µg /kg body weight.

### EXAMPLES

Biopsies were taken from patients suffering from severe asthma bronchiale, after informal consent was given, using the surgical method of bronchoscopy. Obtained tissue probes were stored and processed for isolation of total RNA in RNA*later*™ (patent pending), an aqueous, non-toxic tissue storage reagent that stabilizes and protects cellular RNA in intact, unfrozen tissue samples.
The dissected tissue (less than 0.5 cm in any one dimension) is submerged in approximately 5 volumes of RNAlater (e.g., a 0.5 g sample requires about 2.5 ml of RNAlater) at room temperature. The solution permeates the cells, stabilizing the RNA. Then, RNA is isolated using the one-step RNA isolation methods, such as TRIzol® Reagent (Life Technologies), following the instructions of the supplier and finally eluted in H₂O.

### Preparation of Labeled cRNA and Hybridization to Microarrays.

Double-stranded cDNA was synthesized out of the isolated patients RNA samples using a cDNA synthesis kit (Superscript; Life Technologies) employing oligo(dT) priming. The resulting cDNA was used for *in vitro* transcription (Ambion T7 Megascript system) in the presence of biotin-11-CTP and biotin-16-UTP (Enzo Diagnostics). A total of 25-50 µg of the cRNA product in buffer [40 mM Tris·acetate (pH 8.1)/100 mM potassium acetate/30 mM magnesium acetate] was fragmented at 94°C for 35 min. It was then used as a hybridization probe from each patient for hybridization as recommended (Affymetrix, Santa Clara, CA).
Aliquots of the hybridization patients cRNA mixtures (10 µg cRNA in 200 µl hybridization mix) were hybridized to a Human Genome U133A Array. Each array was washed and scanned (Hewlett Packard, GeneArray scanner G2500A) according to procedures developed by manufacturer (Affymetrix).

**Analysis of GENECHIP Data.** Scanned output files were visually inspected for hybridization artifacts and then analyzed with GENECHIP 3.1 software (Affymetrix).
The expression analysis files created by GENECHIP 3.1 software were transferred to a database (Microsoft Access) and linked to Internet genome databases (e.g., NHLBI, or Swiss Prot).

**Analysis of Infectious agents in biopsies.** As patients with asthma bronchiale repeatedly suffer from inflammations, biopsied material was tested for the presence of infectious agents. Most dominant was the observation of underlying Herpes virus infections (treated with Ganciclovir) and various bacterial infections (treated with various antibiotics), or few fungal infections.

**Therapy.** Therapy with interferon gamma was only started after successful pretreatment of the underlying infections and the assessment of the gene expression of the endogenous disease signiture genes. Application of molecular gene expression analysis followed by interferon gamma, led to surprisingly positive results. In the clinical course two patients were treated who suffered from severe and life-threatening bronchial asthma, non-responsive to oral treatment with prednisolone. In both patients, IFN-γ was used in an attempt to save the patients' lives. Meanwhile, the first patient has used IFN-γ for a period of more than 24 months. After 6 months, lung function of both patients (as assessed by repeated measurement of full lung function and continuous peak flow observation) has fully recovered and remained stable. The actual treatment of first patient consists only of interferon gamma (100 µg, given 3-times subcutaneously per week) and 50 mg of salmeterol, a long acting β₂-agonist. The patient has not experienced any serious infection or asthma attack throughout the observation period of 24 months.
Those data indicate that the pharmaceutical composition of gene expression analysis followed by interferon gamma is able to counterbalance fibrosing reactions in asthma bronchiale and provides significant survival benefit to the patients with asthma bronchiale.

## Claims

1. A pharmaceutical composition comprising
(i) interferon gamma (IFN-γ), optionally together with a pharmaceutically acceptable carrier, diluent or excipient
(ii) a polynucleotide library for the molecular characterization of asthma bronchiale, said library comprising a pool of polynucleotide sequences or subsequences thereof wherein said sequences or subsequences are either underexpressed or overexpressed in asthma bronchiale cells compared to non-asthma bronchiale cells
(iii) a polynucleotide library for the molecular characterization of infectious agents, said library comprising a pool of polynucleotide sequences or subsequences thereof wherein said sequences or subsequences are characteristic for bacteria, mycoplasma, viruses, fungi, or yeasts.

2. The pharmaceutical composition according to claim 1, comprising additionally a glucocorticoid compound or a long acting β₂-agonist.

3. The pharmaceutical composition comprising
(i) pegylated interferon gamma (PEG-IFN-γ), optionally together with a pharmaceutically acceptable carrier, diluent or excipient
(ii) a polynucleotide library for the molecular characterization of asthma bronchiale, said library comprising a pool of polynucleotide sequences or subsequences thereof wherein said sequences or subsequences are either underexpressed or overexpressed in asthma bronchiale cells compared to non-asthma bronchiale cells
(iii) a polynucleotide library for the molecular characterization of infectious agents, said library comprising a pool of polynucleotide sequences or subsequences thereof wherein said sequences or subsequences are characteristic for bacteria, mycoplasma, viruses, fungi, or yeasts.

4. The pharmaceutical composition according to claim 3, comprising additionally a glucocorticoid compound or a long acting β₂-agonist.

5. The pharmaceutical composition according to claims 1-4 for the treatment of asthma bronchiale.

6. The pharmaceutical composition according to claims 1-4 for the treatment of corticosteroid resistant asthma bronchiale.

7. A polynucleotide library according to claims 1-4 wherein said sequences are useful in classifying good and poor prognosis in asthma bronchiale patients.

8. A polynucleotide library according to claims 1-4 wherein said polynucleotide sequences or subsequences correspond to any combination of at least one polynucleotide selected among those included in at least 50%, preferably 75% and more preferably 100% of the predefined sets.

9. A polynucleotide library according to claims 1-4 wherein said polynucleotides are immobilized on a solid support in order to form a polynucleotide array.

10. A polynucleotide library according to claims 1-4 wherein the support is selected from the group comprising a nylon membrane, nitrocellulose membrane, glass slide, glass beads, membranes on glass support or a silicon chip.

11. A polynucleotide library according to claims 1-4 wherein the presence of or predisposition for asthma bronchiale using expression profiling images comprises the steps of:
(a) isolation of material from an individual clinically diagnosed for a lung disease, or of familiar predisposition for asthma bronchiale,
(b) providing molecular probes as ligands selected from the group consisting of mRNA, polynucleotides, oligonucleotides, cDNAs, proteins or functional fragments thereof, isolated or generated from said individuals
(c) contacting said molecular probes as ligands with a set of immobilized receptors selected from the group consisting of polynucleotides, oligonucleotide or cDNA probes and antibodies or functional fragments thereof, specifically representing a respective set of genes involved with the presence of or predisposition for asthma bronchiale, or an infection to be molecularly diagnosed, and qualitatively and quantitatively detecting the presence of bound ligand/receptor complexes to obtain an expression profiling image being representative for the current status of the individual to be diagnosed,
(d) comparing the expression profiling image obtained in step (c) with the expression profiling image(s) of normal individuals, expression profiling image(s) of individuals with clinically similar diseases, and/or with the expression profiling images(s) of individuals having a predisposition for or suffering from asthma bronchiale to be diagnosed, and
(e) excluding or diagnosing suspected asthma bronchiale or a predisposition therefore on the basis of the results of the comparison obtained in step (d).
The pharmaceutical composition according to claims 1-4 wherein said polynucleotide library for the molecular characterization of asthma bronchiale comprises a pool of polynucleotide sequences or subsequences selected from a set of sequences defined by, or correlated to, or derived from the group of genes consisting of candidate genes indicated in the following list:
connective tissue growth factor, M92934.1
transforming growth factor β₁, NM_000660
interferon-gamma, X87308
interleukin-13
Alu-Sq subfamily; U14573
mRNA clone 9112; X57348
prostate differentiation factor; AF003934.1
G protein-coupled receptor 87; NM_023915.1
PROCOLLAGEN ALPHA 1(III) CHAIN PRECURSOR; AU144167
hypothetical protein FLJ13556; BE300252
Tubulin, alpha; AL581768
collagen, type III, alpha 1; AF130082.1
tubulin alpha 1; BC006481.1
lectin, galactoside-binding, soluble, 2 (galectin 2); M87842.1
eukaryotic translation elongation factor 1 alpha 1-like 14; NM_001403.1
eukaryotic translation elongation factor 1 alpha 1; NM_001402.1
annexin A8; NM_001630.1
collagen, type I, alpha 2; NM_000089.1
collagen, type I, alpha 1; NM_000088.1
hexabrachion (tenascin C, cytotactin); M55618.1
ribosomal protein L41; NM_021104.1
insulin-like growth factor binding protein 7; L19182.1
karyopherin alpha 2 (RAG cohort 1, importin alpha 1); U28386.1
vesicle-associated membrane protein 3 (cellubrevin); BC003570.1
TIA1 cytotoxic granule-associated RNA-binding protein; M77142.1
fatty-acid-Coenzyme A ligase, long-chain 2; D10040.1
protein tyrosine phosphatase, non-receptor type 12; D13380.1
transcrption factor IIIA-interacting protein; NM_021980.1
a disintegrin and metalloproteinase domain 10; AF009615.1
tumor necrosis factor (ligand) superfamily, member 10; U37518.1
A kinase (PRKA) anchor protein 2; AB023137.1
lysosomal-associated membrane protein 2; J04183.1
3-phosphoadenosine 5-phosphosulfate synthase 2; AF150754.2
caveolin 2; BC005256.1
membrane metallo-endopeptidase (neutral endopeptidase, enkephalinase, CALLA, CD10); J03779.1
ATP-binding cassette, sub-family A (ABC1), member 1; AF165281.1
adenylate cyclase 7; D25538.1
prenylcysteine lyase; AF181490.1
nebulette; NM_006393.1
KIAA0161 gene product; D79983.1
KIAA0680 gene product; AB014580.1
malic enzyme 1, NADP(+)-dependent, cytosolic; NM_002395.2
Friend leukemia virus integration 1; BC001670.1
G protein-coupled receptor kinase 5; L15388.1
frizzled (Drosophila) homolog 1; AB017363.1
endothelin receptor type A; L06622.1
alpha integrin binding protein 63; AB023234.1
dihydropyrimidine dehydrogenase; U20938.1
Wnt inhibitory factor-1; AF122922.1
KIAA0716 gene product; AB018259.1
retinitis pigmentosa 2; NM_006915.1
E3 ubiquitin ligase SMURF2; AF301463.1
oxidative 3 alpha hydroxysteroid dehydrogenase; retinol dehydrogenase; 3-hydroxysteroid epimerase (RODH); NM_003725.1
integrin, alpha 4 (antigen CD49D, alpha 4 subunit of VLA-4 receptor); NM_000885.2
klotho; AB005142.1
carboxypeptidase M; NM_001874.1
toll-like receptor 3; U88879.1
interleukin 18 (interferon-gamma-inducing factor); D49950.1
interleukin 18 receptor 1; U43672.1
ubiquitin specific protease 9; NM_004654.2
TXK tyrosine kinase; L27071.1
Ste20-related serine/threonine kinase; D86959.1
Sciellin; AF045941.1
pleiomorphic adenoma gene-like 1; U81992.1
UDP-N-acetylglucosamine:polypeptide-N-acetylglucosaminyl transferase; U77413.1
nuclear receptor coactivator 3; AF036892.1
glycoprotein M6B; AF016004.1
TRAF family member-associated NFKB activator; U59863.1
GATA-binding protein 6; U66075.1
potassium inwardly-rectifying channel, subfamily J, member 15; U73191.1
RecQ protein-like (DNA helicase Q1-like); BC001052.1
tissue factor pathway inhibitor (lipoprotein-associated coagulation inhibitor); AF021834.1
calcitonin receptor-like; U17473.1
transcription factor 8 (represses interleukin 2 expression); U12170.1
UDP-Gal:betaGlcNAc beta 1,3-galactosyltransferase, polypeptide 3; AB050856.1
transposon-like element; AL567779
DKFZp434I0812; AI057093
sperm associated antigen 9; AK023512.1
protein tyrosine phosphatase, receptor type, C; AI809341
KIAA0033 protein; AU153138
DKFZP564M1416 protein; BF966021
KIAA0197 protein; D83781.1
hypothetical protein FLJ22028; AI814728
hypothetical protein DKFZp762B226; AK001821.1
Hmob33 protein; AI377497
synaptojanin 1; AB020717.1
Highly similar to A59252 myosin heavy chain, nonmuscle; AI382123
clone 23664 and 23905 sequence; AI769688
DKFZp586B211; AA045174
Novel human gene mapping to chomosome 13; N80918
chloride intracellular channel 2; NM_001289.2
ATPase, Class VI, type 11A; BF439472
KIAA0580 protein; AB011152.1
DKFZp586F1323; AI743740
v-ral simian leukemia viral oncogene homolog A (ras related); M29893.1
synaptosomal-associated protein, 23kD; Y09568.1
collagen, type IV, alpha 3 (Goodpasture antigen); NM_000091.1
hypothetical protein KIAA1164; AU121431
hypothetical protein FLJ20275; AW270932
Homo sapiens mRNA from chromosome 5q21-22, clone FBR89; AB002438.1
KIAA1043 protein; AB028966.1
fragile X mental retardation 1; AA830884
HEMBA1003278; AU144887
KIAA1033 protein; AK001657.1
up-regulated by BCG-CWS; AL049963.1
clone COL05634; AK025152.1
clone LNG01738; AK026679.1
collagen, type IV, alpha 3 (Goodpasture antigen) alternatively spliced mRNA; U02519.1
T cell receptor delta; X06557.1
Highly similar to IFT2 HUMAN INTERFERON-INDUCED PROTEIN WITH TETRATRICOPEPTIDE REPEATS 2; BE888744
CLONE=IMAGE:1269464; AA721025
CLONE=IMAGE:1680846; AI086530
CLONE=IMAGE:3859303; BF032808
hypothetical protein FLJ12910; NM_024573.1
hypothetical protein FLJ13164; NM_022776.1
hypothetical protein FLJ20331; NM_017768.1
hypothetical protein FLJ22029; NM_024949.1
pleckstrin homology domain-containing, family A (phosphoinositide binding specific) member 1; AF286160.1
zinc finger protein 331; AF251515.2
hypothetical protein FLJ14281; NM_024920.1
hypothetical protein FLJ23516; NM_024539.1
hypothetical protein FLJ22690; NM_024711.1
dickkopf (Xenopus laevis) homolog 2; AF177395.1
clone=IMAGE-2075682; AI832249
clone=IMAGE-290072; N64681
clone=IMAGE-2112146; AI400621
KIAA0823 protein; AB020630
protein-tyrosine-phosphatase D1; X79510
lok for protein kinase; AB015718
oxidative 3 alpha hydroxysteroid dehydrogenase; U89281
KIAA0615 protein; AB014515
KIAA1466 protein; AI765383
CLONE=IMAGE:2337182; AI694562
hypothetical protein FLJ20059; AW006750
sec61 homolog; AI719730
clone 24421 mRNA sequence; BE881590
KIAA1598 protein; AU157109
mannosidase, alpha, class 1A, member 1; BG287153
Claudin-18; AF221069.1
colon carcinoma related protein; AF099505.1
hypothetical protein FLJ23151; NM_024772.1
serumglucocorticoid regulated kinase-like; AF169035.1
hyaluronic acid receptor; AF127670.2
hypothetical protein FLJ20716; NM_017938.1
cytochrome P450 retinoid metabolizing protein; AF252297.1
hypothetical protein FLJ23045; NM_024704.1
TMTSP for transmembrane molecule with thrombospondin module; AB044385.1
macrophage scavenger receptor 1; AI299239
and allelic variants or mutants thereof,
and sequences specific for infectious agents, comprising a pool of polynucleotide sequences or subsequences thereof wherein said sequences or subsequences are characteristic for Mycobacterium spec., Mycoplasma spec., Staphyllococcus aureus, Streptococcus spec., Borrelia, Treponema pallidum, Leptospira interrogans, Campylobacter jejuni, Escherichia coli, EPEC, ETEC, EIEC, EHEC Salmonella enterica, Yersinia enterocolitica, Aeromonas spec., Campylobacter fetus, Moraxella catarrhalis, Moraxella catarrhalis, Brucella spec., Toxoplasma, Salmonella enterica, Shigella spec., Yersinia enterocolitica, Vibrio cholerae, Pseudomonas aeruginosa, Burkholderia cepacia, Stenotrophomonas maltophilia, Acinetobacter baumanii, Acitenobacter calcoaceticus, Klebsiella, Enterobacter, Citrobacter, Proteus, Serratia, Morganella, Providencia, Cardiobacterium hominis, Eikenella corrodens, Gardnerella vaginalis, Calymmatobacterium granulomatis, Bacteriodes, Porphyromonas, Prevotella, Fusobacterium, Rickettsia prowazekii, Bartonella bacilliformis, Bartonella henselae, Chlamydia spec., yeasts, fungi, retroviruses, adenoviruses, hepadnaviruses, herpesviruses, influenza viruses, paramyxoviruses.

12. Use of a pharmaceutical kit comprising
(i) a package comprising IFN-γ,
(ii) a package comprising a polynucleotide library for the molecular characterization of asthma bronchiale, said library comprising a pool of polynucleotide sequences or subsequences thereof wherein said sequences or subsequences are either underexpressed or overexpressed in asthma bronchiale cells compared to non-asthma bronchiale cells
(iii)a package comprising a polynucleotide library for the molecular characterization of infectious agents, said library comprising a pool of polynucleotide sequences or subsequences thereof wherein said sequences or subsequences are characteristic for bacteria, mycoplasma, viruses, fungi, or yeasts.

13. Use of pharmaceutical kit according to claim 12 further comprising a package comprising a glucocorticoid compound or a long acting β₂-agonist.

14. Use of a pharmaceutical kit comprising
(i) a package comprising pegylated IFN-γ,
(ii) a package comprising a polynucleotide library for the molecular characterization of asthma bronchiale, said library comprising a pool of polynucleotide sequences or subsequences thereof wherein said sequences or subsequences are either underexpressed or overexpressed in asthma bronchiale cells compared to non-asthma bronchiale cells
(iii)a package comprising a polynucleotide library for the molecular characterization of infectious agents, said library comprising a pool of polynucleotide sequences or subsequences thereof wherein said sequences or subsequences are characteristic for bacteria, mycoplasma, viruses, fungi, or yeasts.

15. Use of pharmaceutical kit according to claim 14 further comprising a package comprising a glucocorticoid compound or a long acting β₂-agonist.

16. Use of pharmaceutical kit according to claims 12-15 for the manufacture of a medicament for the treatment of asthma bronchiale.

17. Use of pharmaceutical kit according to claims 12-15 for the manufacture of a medicament for the treatment of glucocorticoid-resistant asthma bronchiale.

18. Use of pharmaceutical kit according to claims 12-17, wherein IFN-γ is administered to the individual in a single dose amount varying from 2.0 to 3.0 µg / kg body weight.

19. Use of pharmaceutical kit according to claims 12-16, 18, wherein the glucocorticoid compound is administered to the individual in a single dose amount varying from 100 to 150 µg / kg body weight.

20. Use of pharmaceutical kit according to claims 12-19, wherein interferon gamma or pegylated interferon gamma is administered to the individual by inhalation.

21. Method for treatment of asthma bronchiale or related forms thereof comprising administering to a individual a pharmaceutical composition according to any of the claims 1 - 4 or a pharmaceutical kit according to any of the claims 12 - 20.

22. Method according to claim 18, wherein IFN-γ is administered to the individual in a single dose amount varying from 2.0 to 3.0 µg / kg body weight.

23. Method according to claims 21 or 22, wherein the glucocorticoid compound is administered to the individual in a single dose amount varying from 100 to 150 µg / kg body weight.
